# EUROPÄISCHE PATENTSCHRIFT

(11) **EP 1 337 281 B1**
(45) Veröffentlichungstag und Bekanntmachung des Hinweises auf die Patenterteilung: **22.03.2006**
(21) Anmeldenummer: 01998368.3
(22) Anmeldetag: 28.11.2001
(51) Int. Cl.: A61L 2/10, A61L 2/14, B08B 7/00, B01J 19/08, H05H 1/24

(54) **VERFAHREN UND VORRICHTUNG ZUR OBERFLÄCHENBEHANDLUNG VON OBJEKTEN**
METHOD AND DEVICE FOR TREATING THE SURFACES OF ITEMS
PROCEDE ET DISPOSITIF POUR LE TRAITEMENT DE SURFACE D'OBJETS

(30) Priorität: 29.11.2000 DE 10059131
(43) Veröffentlichungstag der Anmeldung: 27.08.2003
(73) Patentinhaber: Fraunhofer-Gesellschaft zur Förderung der angewandten Forschung e.V., 80686 München (DE)
(72) Erfinder: NEFF, Willi, B-4721 Kelmis (BE); TROMPETER, Franz-Josef, 52072 Aachen (DE); FRANKEN, Oliver, 52249 Eschweiler (DE); POCHNER, Klaus, 65428 Rüsselsheim (DE)
(74) Vertreter: Gagel, Roland
(86) Internationale Anmeldenummer: PCT/DE2001/004484
(87) Internationale Veröffentlichungsnummer: WO 2002/043781

(56) Entgegenhaltungen:
- EP-A- 0 948 969
- DE-A- 4 332 866
- DE-C- 4 302 465
- US-A- 4 837 484

## Beschreibung

### Technisches Anwendungsgebiet

Die vorliegende Erfindung betrifft ein Verfahren sowie eine Vorrichtung zur Oberflächenbehandlung von Objekten, insbesondere von bahnförmigem oder tiefgezogenem Material, bei dem die zu behandelnde Oberfläche des Objektes in einem mit einem ersten Gas oder Gasgemisch befüllten Entladungsraum einer Barriereentladung ausgesetzt wird, die zwischen einer ersten und einer zweiten flächigen Elektrode erzeugt wird.

Die Behandlung von Oberflächen, insbesondere deren Reinigung, Entkeimung, Sterilisation, Desinfektion oder Aktivierung, spielt in vielen technischen Bereichen eine wesentliche Rolle. So muss bspw. bahnförmiges Packmaterial vor der weiteren Verwendung an der Oberfläche entkeimt oder sterilisiert werden. Eine derartige Entkeimung oder Sterilisation lässt sich beispielsweise mit dem vorliegenden Verfahren und der vorliegenden Vorrichtung in vorteilhafter Weise durchführen.

### Stand der Technik

Aus der DE 41 13 524 A1 sowie der EP 510 503 A2 sind Verfahren und Vorrichtungen für die Reinigung von Oberflächen bekannt. In beiden Fällen ist eine Hochleistungs-Entladungsröhre deutlich beabstandet von einem zu reinigenden Substrat vorgesehen. Das Substrat wird im ersten Fall von der UV-Bestrahlung photochemisch verändert, um Beschichtungsmaterial besser zu verankern. Im zweiten Fall des Standes der Technik werden durch die UV-Bestrahlung Radikale gebildet. Die UV-Strahlung wird durch eine Barriereentladung in der Hochleistungs-Entladungsröhre erzeugt. Eine derartige Barriereentladung, in der Literatur auch als dielektrisch behinderte Entladung oder stille Entladung bezeichnet, tritt in einem zwischen zwei Elektroden gebildeten Entladungsraum, von denen zumindest eine Elektrode vom Entladungsraum durch eine dielektrische Barriere getrennt ist, dann auf, wenn die zündspannung bzw. die Zündfeldstärke im Entladungsraum überschritten wird. Je nach Druckbereich und Gaszusammensetzung bildet sich ein homogenes Plasma aus oder es entstehen dünne Ladungskanäle, sog. Filamente, die jeweils nur für wenige Nanosekunden existieren. Durch derartige Barriereentladungen wird bei Einsatz eines geeigneten Gases im Entladungsraum UV-Strahlung hoher Intensität freigesetzt, so dass derartige Vorrichtungen als UV-Hochleistungsstrahler eingesetzt werden können. Hierzu müssen jedoch zumindest eine der Elektroden sowie das Dielektrikum für UV-Strahlung transparent sein.

Die DE 43 02 465 C1 zeigt eine Vorrichtung, bei der eine der Elektroden durch ein spannungsangeregtes Plasma in einem Gas gebildet wird, dessen Druck um mindestens zwei Potenzen niedriger ist, als der Gasdruck im Entladungsraum. Das Gas des als Elektrode eingesetzten spannungsangeregten Plasmas ist in einer Kammer aus einem dielektrischen Material eingeschlossen, dessen senkrecht zur ersten Elektrode verlaufende Seiten eine oder mehrere weitere Elektroden zur Anregung dieses Niederdruckplasmas aufweisen. Das dielektrische Material der Kammer ist für UV-Strahlung durchlässig und bildet gleichzeitig die dielektrische Barriere im Entladungsraum. Das Gas in der Kammer ist derart gewählt, dass es, insbesondere im plasmaangeregtem Zustand, für die im Entladungsraum erzeugte UV-Strahlung transparent ist. Auf diese Weise wird eine für UV-Strahlung transparente Elektrode realisiert. Die in dieser Druckschrift angeführten Anwendungen der im Entladungsraum erzeugten UV-Strahlung beziehen sich auf die Induzierung chemischer Reaktionen, die Anregung von Farbstoffen sowie die Homogenisierung von Mittel- und Hochdruckplasmen in Lasern und bei der plasmaunterstützten Stoffabscheidung aus der Gasphase.

Aus der DE 43 32 866 C2 sind ein Verfahren sowie eine Vorrichtung zur Oberflächenbehandlung von Objekten bekannt, bei denen die zu behandelnde Oberfläche des Objektes in einem mit einem ersten Gas befüllten Entladungsraum einer Barriereentladung ausgesetzt wird, die zwischen einer ersten und einer zweiten flächigen Elektrode erzeugt wird. Zu behandelndes bahnförmiges Material bildet hierbei direkt die dielektrische Barriere zwischen einer der Elektroden und dem Entladungsraum. In einer weiteren Ausgestaltung des offenbarten Verfahrens wird das Objekt außerhalb des Entladungsraums in unmittelbarer Nachbarschaft der als Gitterelektrode ausgebildeten zweiten Elektrode angeordnet, so dass die Barriereentladung durch die Gitterelektrode hindurch auf die Oberfläche des Objektes wirken kann. Durch die direkte Einwirkung der Barriereentladung wird eine Reinigung der Oberfläche aufgrund plasmachemischer Zersetzung erreicht.

In einer weiteren in dieser Druckschrift offenbarten Ausgestaltung ist der Entladungsraum zwischen einer ersten flächigen Elektrode und einer mit einem Gas befüllten Kammer gebildet, die aus einem für UV-Strahlung durchlässigen dielektrischen Material besteht. Diese aus der DE 43 02 465 C1 bekannte Vorrichtung wird als UV-Strahler betrieben, wobei die zu behandelnde Oberfläche hier außerhalb des Entladungsraums mit der durch die zweite Elektrode hindurch tretenden UV-Strahlung beaufschlagt wird. Durch die Einwirkung dieser im Entladungsraum erzeugten UV-Strahlung wird durch photochemische Prozesse ebenfalls eine Reinigung der bestrahlten Oberfläche erreicht.

Ausgehend von diesem Stand der Technik besteht die Aufgabe der vorliegenden Erfindung darin, ein Verfahren sowie eine Vorrichtung zur Oberflächenbehandlung von Objekten anzugeben, die eine höhere Effizienz sowie eine Steigerung der Prozessgeschwindigkeit bei der Oberflächenbehandlung ermöglichen. Insbesondere sollen die Vorrichtung und das Verfahren eine schnelle Entkeimung von Oberflächen, insbesondere von bahnförmigen Materialien, sowie eine vollständige Sterilisation die bisher mit einer UV-Behandlung nicht erreichbar ist, ermöglichen.

### Darstellung der Erfindung

Die Aufgabe wird mit dem Verfahren und der Vorrichtung gemäß den Patentansprüchen 1 bzw. 12 gelöst. Vorteilhafte Ausgestaltungen des Verfahrens sowie der Vorrichtung sind Gegenstand der Unter-ansprüche. Anspruch 22 gibt schließlich noch eine alternative Betriebsweise der erfindungsgemäßen Vorrichtung zur Behandlung von Oberflächen an.

Bei dem vorliegenden Verfahren wird die zu behandelnde Oberfläche des Objektes in einem mit einem ersten Gas oder Gasgemisch befüllten Entladungsraum einer Barriereentladung ausgesetzt, die zwischen einer ersten und einer zweiten flächigen Elektrode erzeugt wird. Als zweite Elektrode wird ein plasmaangeregtes zweites Gas oder Gasgemisch eingesetzt, das UV-Strahlung emittiert. Insbesondere wird dieses zweite plasmaangeregte Gas oder Gasgemisch vorzugsweise ebenfalls über eine Barriereentladung angeregt. Durch diese zweistufige Entladung, einerseits die Barriereentladung des ersten Gases oder Gasgemisches im Entladungsraum und andererseits die Entladung bzw. Barriereentladung des zweiten Gases oder Gasgemisches, wird eine effiziente und schnelle Oberflächenbehandlung erreicht. Die direkte Einwirkung der Barriereentladung im Entladungsraum, in der das Objekt angeordnet oder durch den es hindurch geführt wird, bewirkt eine plasmachemische Oberflächenbehandlung durch Radikale, während gleichzeitig durch das als zweite Elektrode dienende plasmaangeregte zweite Gas bzw. die Barriereentladung in diesem Gas eine intensive UV-Bestrahlung der Oberfläche erzielt wird.

Die zweite Elektrode kann hierbei ähnlich wie die durch ein plasmaangeregtes Gas gebildete zweite Elektrode der DE 43 02 465 C1 ausgestaltet sein. Während bei dieser Druckschrift die homogenisierende Wirkung und die UV-Transparenz der Elektrode ausgenutzt werden, werden bei dem vorliegenden Verfahren Gase oder Gasgemische, wie bspw. Edelgase oder Edelgashalogenidgemische, in die für das zweite Gas vorgesehene Kammer eingefüllt, die selbst in der darin stattfindenden Barriereentladung effektiv UV-Strahlung erzeugen. Diese stark UV-strahlende Gasentladung stellt gleichzeitig die zweite Elektrode für die direkt auf die zu behandelnde Oberfläche wirkende Barriereentladung des Entladungsraums dar. Die im Folgenden als Plasmaelektrode bezeichnete zweite Elektrode ist damit von der direkten Gasentladung auf der Oberfläche des Objekts getrennt und kann auch im Über- oder im Unterdruck, bspw. bei 500*10² Pa (500 mbar), betrieben werden. Die wesentlich nähere und direktere UV-Exposition der zu behandelnden Oberfläche ohne eine abschattende Metallelektrode sowie die gleichzeitige Behandlung durch die zweite direkte Barriereentladung verbessern die Effizienz der Oberflächenbehandlung, insbesondere die Reinigungswirkung oder entkeimende Wirkung auf die Oberfläche. Über die Qualität einer reinen UV-Behandlung hinaus ist durch die zusätzliche plasmachemische Wirkung auch eine vollständige Sterilisation und damit die Anwendung im aseptischen Verpackungsbereich bei Temperaturen < 70°C möglich.

Vorzugsweise wird das plasmaangeregte zweite Gas oder Gasgemisch unter einem Druck von zumindest 100*10² Pa (100 mbar) gesetzt. Durch geeignete Wahl dieses zweiten Gases oder Gasgemisches kann eine starke und optimierte UV- und VUV-Emission erreicht werden. Hierfür sind insbesondere bekannte Excimergase, wie bspw. Xe oder KrCl, geeignet.

Das Gas im Entladungsraum kann bspw. Luft oder feuchte Luft sein, die unter Atmosphärendruck steht. Vorzugsweise werden jedoch während der Behandlung Gase, Gasgemische oder Dämpfe zusätzlich in den Entladungsraum eingeleitet, die eine Verbesserung der für die Oberflächenbehandlung beabsichtigten Wirkung herbeiführen. So kann bspw. eine Verbesserung der Entkeimungswirkung durch verschiedene, die Entkeimung begünstigende Wirkmechanismen hervorgerufen werden. Ein Beispiel ist die Erhöhung der UV-Emission in der Barriereentladung des Entladungsraumes durch Zuführen von Argon oder Stickstoff oder durch die Zumischung von Wasserstoff. Ebenso wird der Einfluss von Teilchenbeschuss, z. B. Ionen, auf die zu reinigende Oberfläche durch die Zumischung leichter Gase, wie bspw. Wasserstoff, erhöht. Eine Verstärkung der Reinigungswirkung, insbesondere der Desinfektion und Sterilisation der Oberfläche, durch zusätzliche chemische bzw. plasmachemische Oxidation wird durch die Zumischung oxidativ wirkender Gaskomponenten, wie bspw. Sauerstoff, Ozon, Wassergas, Wasserdampf, Wasserstoffperoxidgas oder - dampf zum Gasgemisch in der Barriereentladung des Entladungsraums erreicht. Darüber hinaus erlaubt eine Zumischung von Edelgasen, wie z. B. Helium oder Argon eine Homogenisierung der Barriereentladung. Durch eine homogenisierte Flächenerfassung der Gasentladung wird die Reinigung, insbesondere die Sterilisation, der Oberfläche verbessert. Der Entladungsraum kann für die zusätzliche Zuführung derartiger Gase tunnelartig ausgebildet sein, so dass die zusätzlich zugeführten Gase, Gasgemische oder Dämpfe die Umgebungsluft verdrängen. Die tunnelartige Ausgestaltung wird durch eine geeignete geometrische Form der Elektroden erreicht.

In einer weiteren vorteilhaften Ausführungsform des vorliegenden Verfahrens wird die Barriereentladung im Entladungsraum zur Erzielung einer höheren Dichte der Entladungsfilamente oder zur Erzielung einer homogenen Gasentladung auf der zu entkeimenden Oberfläche gepulst angeregt. Diese gepulste Anregung, wie sie bspw. aus der DE 196 43 925 A1 bekannt ist, erfolgt durch Beaufschlagung der Elektroden mit steilen Spannungsanstiegen, wodurch die Zündfeldstärke der Entladungsfilamente erhöht wird. Bei Spannungsanstiegen ab 1 kV/µs - bei Atmosphärendruck besser 10kV/ns - werden die Homogenität der Filamentierung sowie die UV-Ausbeute in beiden Gasentladungen deutlich erhöht. Die hiermit verbundene bessere Oberflächenerfassung der Entladungsfilamente verbessert die Wirkung und die Effizienz der Reinigung.

Vorzugsweise werden bei der Durchführung des vorliegenden Verfahrens großflächige plane Elektroden eingesetzt, so dass gleichzeitig eine größere Oberfläche mit der Barriereentladung und der UV-Strahlung beaufschlagt wird. Auch eine Anordnung mehrerer derartiger Elektroden hinter- und/oder nebeneinander zur Abdeckung einer größeren Oberfläche bietet Vorteile, insbesondere eine Erhöhung der Prozessgeschwindigkeit.

Bei der Oberflächenbehandlung von bahnförmigem Material wird dieses vorzugsweise zwischen Plasmaelektrode und geerdeter Elektrode durch den Entladungsraum bewegt. In Transportrichtung dieses bahnförmigen Materials können hierbei wiederum mehrere derartige Elektrodenpaare angeordnet sein, um gleichzeitig eine größere Oberfläche mit Barriereentladungen und UV-Strahlung beaufschlagen zu können.

Die vorliegende Vorrichtung weist einen Entladungsraum auf, der zwischen einer ersten flächigen Elektrode und einer mit einem Gas oder Gasgemisch gefüllten, vorzugsweise geschlossenen, Kammer gebildet ist, die zumindest auf einer zur ersten Elektrode gerichteten ersten Seite aus einem für UV-Strahlung durchlässigen dielektrischen Material besteht. Die Kammer grenzt auf einer von der ersten Elektrode abgewandten zweiten Seite an eine weitere flächige Metallelektrode oder wird durch diese abgeschlossen, und ist mit einem im plasmaangeregten Zustand UV-Strahlung emittierenden Gas befüllt. Zum Betrieb der Vorrichtung werden die erste und die weitere Elektrode mit einer Wechselspannung bzw. gepulsten Spannung beaufschlagt, die zur Zündung der beiden Plasmen führt.

Im Gegensatz zu einer Vorrichtung wie die der DE 43 32 866 C2 oder der DE 43 02 465 C1 ist somit keine Elektrode an einer Seitenwandung der Kammer senkrecht zur ersten oder zweiten Seite vorgesehen. Auch die Befüllung der Kammer mit UV-Strahlung emittierendem Gas bei Plasmaanregung und der höhere Druck des Gases in der Kammer unterscheiden die vorliegende Vorrichtung von den bekannten Vorrichtungen. Vorzugsweise beträgt der Druck des Gases in der Kammer zumindest 100*10² Pa (100 mbar), kann jedoch auch deutlich darüber liegen.

Vorzugsweise sind die erste und die weitere Elektrode sowie die erste und zweite Seite der Kammer plan und parallel zueinander ausgebildet. Das dielektrische Material der Kammer kann hierbei bspw. aus Quarzglas bestehen. Die zweite Seite der Kammer kann entweder ebenfalls aus Quarzglas bestehen oder direkt durch die weitere Elektrode gebildet sein. Selbstverständlich kann auch die erste Elektrode als Plasmaelektrode, d. h. in Form eines plasmaangeregten Gases in einer entsprechenden Kammer ausgebildet sein.

Für die Bearbeitung von nicht planen Objekten, beispielsweise von tiefgezogenen Objekten, können die Elektroden auch eine entsprechend an die Form der Objekte angepasste dreidimensionale Form aufweisen.

Die vorliegende Vorrichtung eignet sich insbesondere für flache bzw. dünne Objekte, da der Abstand zwischen der ersten Seite der Kammer und der ersten Elektrode in der Regel im Bereich zwischen ein und fünf Millimeter liegt, so dass nur entsprechend dünne Materialien durch diesen Entladungsraum hindurch geführt oder in diesem Entladungsraum angeordnet werden können. Bei der Oberflächenbearbeitung von bahnförmigem Material wird dieses kontinuierlich oder schrittweise durch den Entladungsraum geführt, während die beiden Entladungen aufrecht erhalten werden. Der Ent-ladungsraum muss hierbei selbstverständlich beidseitig Öffnungen für die Zuführung dieses bahnförmigen Materials aufweisen.

Das vorliegende Verfahren und die vorliegende Vorrichtung lassen sich insbesondere zur Reinigung, Entkeimung, Sterilisierung, Desinfektion oder Aktivierung von Oberflächen einsetzen. Eine besonders vorteilhafte Anwendung betrifft die Entkeimung von bahnförmigem Packmaterial, die durch das vorliegende Verfahren und die zugehörige Vorrichtung mit hoher Geschwindigkeit und Effizienz durchgeführt werden kann. Eine weitere vorteilhafte Anwendung betrifft die Reinigung von Wafern, insbesondere die Feinst-Reinigung oder Entfettung. Auch eine Folienvorbehandlung oder Oberflächenaktivierung von Folien lässt sich mit dem vorliegenden Verfahren und der zugehörigen Vorrichtung sehr vorteilhaft durchführen.

Die vorliegende Vorrichtung lässt sich auch in einer Weise betreiben, bei der an die erste und weitere Elektrode lediglich die Zündspannung angelegt wird, bei der das Plasma in der Kammer zündet, im Entladungsraum unter Atmosphärendruck jedoch nicht. Auf diese Weise wird ein UV-Flachstrahler ohne abschattende Netzelektrode realisiert, über den die zu behandelnde Oberfläche in unmittelbarer Nähe mit UV-Strahlung beaufschlagt wird, um eine photochemische Oberflächenbehandlung zu bewirken.

### Kurze Beschreibung der Zeichnungen

Das vorliegende Verfahren sowie die zugehörige Vorrichtung werden nachfolgend anhand von Ausführungsbeispielen in Verbindung mit den Zeichnungen nochmals kurz erläutert. Hierbei zeigen:
- Fig. 1: ein Beispiel für den Aufbau der vorliegenden Vorrichtung sowie deren Betrieb;
- Fig. 2: ein weiteres Beispiel für die Ausgestaltung der vorliegenden Vorrichtung und deren Betrieb; und
- Fig. 3: ein drittes Beispiel für die Ausgestaltung der vorliegenden Vorrichtung und deren Betrieb.

### Wege zur Ausführung der Erfindung

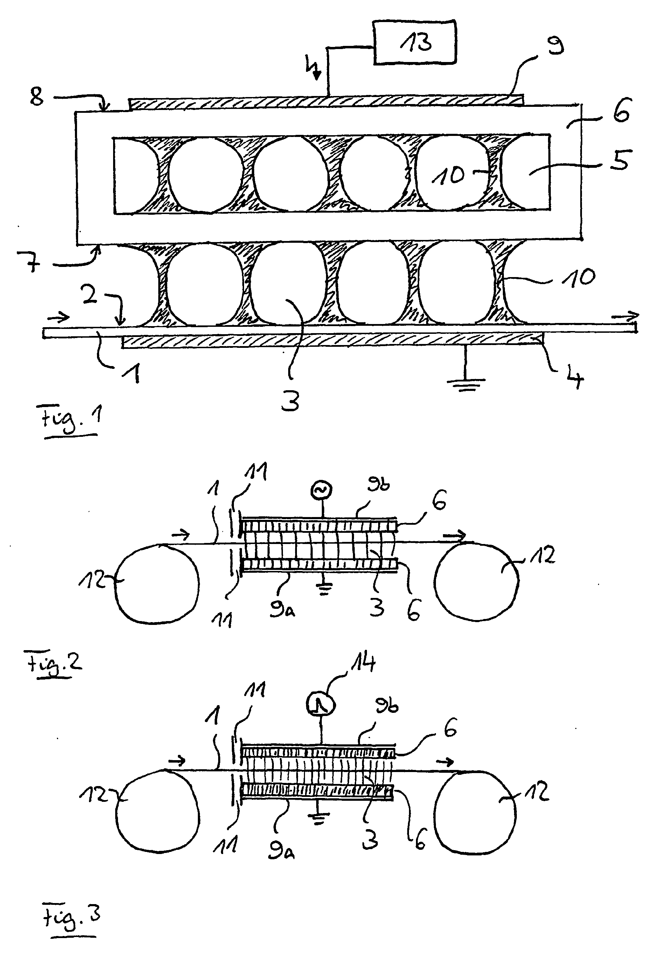

Figur 1 zeigt ein Beispiel für eine Ausgestaltung der vorliegenden Vorrichtung sowie deren Betriebsweise. In der Figur ist der Entladungsraum 3 zu erkennen, der zwischen einer ersten flächigen Elektrode 4 und einer Kammer 6 aus einem UV-transparenten Dielektrikum gebildet ist. Auf der der ersten Elektrode 4 abgewandten Seite 8 der Kammer 6 ist eine weitere Elektrode 9 angeordnet, die mit Hochspannung aus einem Hochspannungsgenerator 13 beaufschlagt wird. Diese Hochspannung liegt typischerweise in einer Größenordnung von ca. 15 kV und wird als Wechselspannung mit 50 Hz bis 200 kHz eingespeist. Die Kammer 6 ist mit einem Excimeredelgas befüllt. Zwischen der ersten Seite 7 der Kammer 6 und der ersten Elektrode 4 befindet sich feuchte Luft im Entladungsraum 3. Das im vorliegenden Fall zu entkeimende Material, in diesem Beispiel eine Kunststofffolie 1, wird nahe der ersten Elektrode 4 in Pfeilrichtung durch den Entladungsraum 3 geführt.

Bei der Oberflächenbehandlung der Kunststofffolie wird durch die zwischen den beiden Elektroden 4, 9 anliegende Hochspannung sowohl in dem Edelgas innerhalb der Kammer 6 als auch in der Luft des Entladungsraumes 3 eine Barriereentladung gezündet, die in der vorliegenden Darstellung durch die angedeuteten Entladungsfilamente 10 veranschaulicht wird. Die Barriereentladung im Entladungsraum 3, im Folgenden als erste Entladung bezeichnet, wirkt hierbei direkt auf die Oberfläche 2 der Kunststofffolie 1, so dass eine plasmachemische Reinigung erzielt wird. Die Barriereentladung innerhalb der Kammer 6, im Folgenden als zweite Entladung bezeichnet, führt aufgrund des gewählten Excimergases zu einer starken UV-Emission, die durch das UV-transparente dielektrische Material der Seite 7 der Kammer 6 hindurch tritt und gleichzeitig mit der ersten Barriereentladung auf die Oberfläche 2 der Kunststofffolie 1 einwirkt, so dass die plasmachemische durch eine photochemische Reinigungswirkung unterstützt wird.

Diese kaskadierte Barriereentladung ermöglicht eine Entkeimung der Oberfläche der Kunststofffolie 1 mit hoher Effizienz und Geschwindigkeit. So konnte bspw. durch Messungen gezeigt werden, dass eine Keimreduktion der Oberfläche einer PET-Folie um 99,999% in weniger als 2 s erreicht wird.

Figur 2 zeigt eine Ausgestaltung der vorliegenden Vorrichtung, bei der zusätzlich Gaszuführungen 11 für die Zuführung von den Oberflächenbehandlungsprozess unterstützenden Gasen am Rand des Entladungsraums 3 zu erkennen sind. In diesem Beispiel ist auch auf der Seite der ersten Elektrode 9a eine weitere Kammer 6 mit einem UV-strahlenden plasmaangeregten Gas angeordnet, die aus einem dielektrischen Material besteht. Der Entladungsraum 3 befindet sich zwischen den beiden Kammern 6, die wiederum an die flächigen Elektroden 9a, 9b angrenzen. Beide Kammern 6 sind in diesem Beispiel identisch ausgestaltet und mit Excimergas befüllt. Bei dieser Anordnung wird die Folie 1 beidseitig von Barriereentladungen und W-Strahlung beaufschlagt, so dass eine beidseitige Oberflächenbehandlung erfolgt. Die Folie 1 wird während der Oberflächenbehandlung über Auf- und Abwickelrollen 12 durch den Entladungsraum 3 geführt.

Figur 3 zeigt schließlich eine Weiterbildung der Vorrichtung nach Figur 2, bei der die Elektroden 9a, 9b mit Hochspannungsimpulsen 14 beaufschlagt werden, so dass eine dichtere Verteilung der Filamente innerhalb der Gasentladungen erreicht wird. Diese dichtere Verteilung der Filamente erhöht die Homogenität der Oberflächenbehandlung und verbessert die UV-Konversionseffizienz der Barriereentladung in Kammer 6, beispielsweise von 30% auf 60% in Xe.

### Bezugszeichenliste

- 1: Objekt
- 2: Oberfläche des Objektes
- 3: Entladungsraum
- 4: erste Elektrode
- 5: zweite Elektrode
- 6: Kammer
- 7: erste Seite der Kammer
- 8: zweite Seite der Kammer
- 9: weitere Elektrode
- 9a: erste Elektrode
- 9b: weitere Elektrode
- 10: Filamente
- 11: Gaszuführungen
- 12: Auf- bzw. Abwickelrolle
- 13: Hochspannungsgenerator
- 14: Hochspannungsimpulse

## Patentansprüche

1. Verfahren zur Oberflächenbehandlung von Objekten, insbesondere von bahnförmigem oder tiefgezogenem Material, bei dem die zu behandelnde Oberfläche (2) des Objektes (1) in einem mit einem ersten Gas oder Gasgemisch befüllten Entladungsraum (3) einer Barriereentladung ausgesetzt wird, die zwischen einer ersten (4) und einer zweiten flächigen Elektrode (5) erzeugt wird,
**dadurch gekennzeichnet,**
**dass** als zweite Elektrode (5) ein plasmaangeregtes zweites Gas oder Gasgemisch eingesetzt wird, das UV-Strahlung emittiert, der die zu behandelnde Oberfläche (2) zusätzlich ausgesetzt wird.

2. Verfahren nach Anspruch 1,
**dadurch gekennzeichnet,**
**dass** das plasmaangeregte zweite Gas oder Gasgemisch unter einem Druck von zumindest 100*10² Pa steht.

3. Verfahren nach Anspruch 1 oder 2,
**dadurch gekennzeichnet,**
**dass** als plasmaangeregtes zweites Gas oder Gasgemisch ein in plasmaangeregtem Zustand Excimere bildendes Gas oder Gasgemisch eingesetzt wird.

4. Verfahren nach einem der Ansprüche 1 bis 3,
**dadurch gekennzeichnet,**
**dass** ein oder mehrere zusätzliche Gase in den Entladungsraum (3) eingeleitet werden, die die Wirkung der Oberflächenbehandlung und/oder die Homogenität der Barriereentladung verbessern.

5. Verfahren nach Anspruch 4,
**dadurch gekennzeichnet,**
**dass** als zusätzliche Gase Helium, Argon, Stickstoff, Wasserstoff, Sauerstoff, Ozon, Wassergas , Wasserdampf, Wasserstoffperoxidgas-oder -dampf oder Kombinationen dieser Gase in den Entladungsraum (3) eingeleitet werden.

6. Verfahren nach einem der Ansprüche 1 bis 5,
**dadurch gekennzeichnet,**
**dass** gepulste Spannungen an die Elektroden (4, 9) angelegt werden.

7. Verfahren nach einem der Ansprüche 1 bis 6,
**dadurch gekennzeichnet,**
**dass** eine planare erste Elektrode (4) eingesetzt wird, die metallisch oder objektseitig mit einer dielektrischen Schicht versehen sein kann.

8. Verfahren nach Anspruch 7,
**dadurch gekennzeichnet,**
**dass** die erste (4) und zweite Elektrode (5) derart großflächig ausgebildet sind, dass sie bei der Oberflächenbehandlung eines bahnförmigen Materials zumindest in einer Dimension eine größere Ausdehnung als die Breite des bahnförmigen Materials aufweisen.

9. Verfahren nach einem der Anspruch 7 oder 8,
**dadurch gekennzeichnet,**
**dass** mehrere erste (4) und zweite Elektroden (5) nebeneinander und/oder hintereinander angeordnet werden, um gleichzeitig einen größeren Oberflächenbereich zu behandeln.

10. Verfahren nach einem der Ansprüche 1 bis 9,
**dadurch gekennzeichnet,**
**dass** Objekt (1) während der Barriereentladung durch den Entladungsraum bewegt wird.

11. Verfahren nach einem der Ansprüche 1 bis 10 zur Reinigung und/oder Desinfektion und/oder Sterilisation und/oder Aktivierung von Oberflächen, insbesondere zur Entkeimung oder Sterilisation von Packmaterial.

12. Vorrichtung zur Oberflächenbehandlung von flachen Objekten, insbesondere von bahnförmigem oder tiefgezogenem Material, mit einem Entladungsraum (3), der zwischen einer ersten flächigen Elektrode (4, 9a) und einer mit einem Gas befüllten Kammer (6) gebildet ist, die zumindest auf einer zur ersten Elektrode (4, 9a) gerichteten ersten Seite (7) aus einem für UV-Strahlung durchlässigen dielektrischen Material besteht,
**dadurch gekennzeichnet,**
**dass** die Kammer (6) auf einer von der ersten Elektrode (4, 9a) abgewandten zweiten Seite (8) an eine weitere flächige Elektrode (9, 9b) angrenzt oder durch diese abgeschlossen wird und mit einem in plasmaangeregtem Zustand UV-Strahlung emittierenden Gas oder Gasgemisch befüllt ist.

13. Vorrichtung nach Anspruch 12,
**dadurch gekennzeichnet,**
**dass** der Druck des Gases bzw. Gasgemisches in der Kammer (6) zumindest 100*10² Pa beträgt.

14. Vorrichtung nach Anspruch 12 oder 13,
**dadurch gekennzeichnet,**
**dass** die erste (4, 9a) und weitere Elektrode (9, 9b) sowie die erste (7) und zweite Seite (8) der Kammer (6) planar ausgebildet sind.

15. Vorrichtung nach Anspruch 12 oder 13,
**dadurch gekennzeichnet,**
**dass** die erste (4, 9a) und weitere Elektrode (9, 9b) sowie die erste (7) und zweite Seite (8) der Kammer (6) dreidimensional, insbesondere gekrümmt, ausgebildet sind.

16. Vorrichtung nach einem der Ansprüche 12 bis 15,
**dadurch gekennzeichnet,**
**dass** der Entladungsraum (3) mit Luft, feuchter Luft, Sauerstoff oder Argon befüllt oder durchströmt ist.

17. Vorrichtung nach einem der Ansprüche 12 bis 16,
**dadurch gekennzeichnet,**
**dass** die Kammer (6) mit einem in plasmaangeregtem Zustand Excimere bildenden Gas oder Gasgemisch, beispielsweise Xe oder KrC1, befüllt ist.

18. Vorrichtung nach einem der Ansprüche 12 bis 17,
**dadurch gekennzeichnet,**
**dass** das dielektrische Material der Kammer (6) Quarzglas, insbesondere ein synthetisches Quarzglas ist.

19. Vorrichtung nach einem der Ansprüche 12 bis 18,
**dadurch gekennzeichnet,**
**dass** zwischen dem Entladungsraum (3) und der ersten Elektrode (9a) eine Kammer (6) aus einem dielektrischen Material angeordnet ist, die mit einem Gas oder Gasgemisch befüllt ist, das in plasmaangeregtem Zustand UV-Strahlung emittiert.

20. Vorrichtung nach einem der Ansprüche 12 bis 19,
**dadurch gekennzeichnet,**
**dass** die erste Elektrode (4, 9a) einen Abstand zwischen 0,1 und 5 mm von der ersten Seite (7) der Kammer (6) aufweist.

21. Vorrichtung nach einem der Ansprüche 12 bis 20,
**dadurch gekennzeichnet,**
**dass** der Entladungsraum (3) auf zumindest zwei Seiten offen ist.

22. Verfahren zum Betrieb einer Vorrichtung nach einem der Ansprüche 12 - 21,
bei dem zwischen der ersten (4, 9a) und weiteren Elektrode (9, 9b) eine Zündspannung angelegt wird, die nur zur Zündung einer Entladung in der Kammer (6), nicht jedoch zur Zündung einer Entladung im Entladungsraum (3) ausreicht, so dass im Entladungsraum (3) angeordnete Objekte nur mit UV-Strahlung aus der Kammer (6) beaufschlagt werden.

## Claims

1. A method for treating the surface of objects, in particular made of strip material or of deep-drawn material, in which the to-be-treated surface (2) of the object (1) is subjected in a discharge region (3) filled with a first gas or gas mixture to a barrier discharge generated between a first planar electrode (4)and a second planar electrode (5),
**characterized in that**
a plasma-excited second gas or gas mixture emitting UV radiation to which said to-be-treated surface (2) is additionally subjected is provided as said second electrode (5).

2. A method according to claim 1,
**characterized in that**
said plasma-excited second gas or gas mixture is under a pressure of at least 100*10² Pa.

3. A method according to claim 1 or 2,
**characterized in that**
a gas or gas mixture forming excimers in a plasma-excited state is provided as said plasma-excited second gas or gas mixture.

4. A method according to one of the claims 1 to 3,
**characterized in that**
one or a multiplicity of additional gases which enhance the effect of the surface treatment and/or the uniformity of the barrier discharge are introduced into said discharge region (3).

5. A method according to claim 4,
**characterized in that**
helium, argon, nitrogen, hydrogen, oxygen, ozone, .water gas, water vapor, hydrogen peroxide gas or hydrogen peroxide vapor or a combination of said gases are introduced into said discharge region (3).

6. A method according to one of the claims 1 to 5,
**characterized in that**
pulsed voltages are applied to said electrodes (4,9).

7. A method according to one of the claims 1 to 6,
**characterized in that**
a planar first electrode (4) is provided which can be provided metallic or with a dielectric layer on the object side.

8. A method according to claim 7,
**characterized in that**
said first electrode (4) and said second electrode (5) are designed in such a large surface manner that when treating the surface of a strip material they expand further in at least one dimension than the width of the strip material.

9. A method according to one of the claims 7 or 8,
**characterized in that**
a plurality of first electrodes (4) and second electrodes (5) are placed side by side and/or behind each other in order to treat a large surface region simultaneously.

10. A method according to one of the claims 1 to 9,
**characterized in that**
said object (1) is moved through said discharge region during the barrier discharge.

11. A method according to one of the claims 1 to 10 to clean and/or disinfect and/or sterilize and/or activate surfaces, in particular to degerminate or sterilize packing material.

12. A device for treating the surface of flat objects, in particular of strip material or of deep-drawn material said device having a discharge region (3) which is formed between a first planar electrode (4, 9a) and a chamber (6) filled with a gas, said chamber (6) being made of a dielectric material permeable for UV radiation on at least one first side (7) facing said first electrode (4, 9a),
**characterized in that**
said chamber (6) borders on a second side (8) facing away from said first electrode (4, 9a) a further planar electrode (9, 9b) or is closed by the same and is filled with a gas or gas mixture emitting UV radiation in a plasma-excited state.

13. A device according to claim 12,
**characterized in that**
the pressure of said gas respectively said gas mixture in said chamber (6) is at least 100*10² Pa.

14. A device according to claim 12 or 13,
**characterized in that**
said first electrode (4, 9a) and said further electrode (9, 9b) as well as said first side (7) and said second side (8) of said chamber (6) are designed planar.

15. A device according to claim 12 or 13,
**characterized in that**
said first electrode (4, 9a) and said further electrode (9, 9b) as well as said first side (7) and said second side (8) of said chamber (6) are designed three-dimensional, in particular curved.

16. A device according to one of the claims 12 to 15,
**characterized in that**
said discharge region (3) is filled with air, moist air, oxygen or argon or air, moist air, oxygen or argon flow through said discharge region (3).

17. A device according to one of the claims 12 to 16,
**characterized in that**
said chamber (6) is filled with a gas or gas mixture which forms excimers in a plasma-excited state, for example Xe or KrCl.

18. A device according to one of the claims 12 to 17,
**characterized in that**
said dielectric material of said chamber (6) is quartz glass, in particular a synthetic quartz glass.

19. A device according to one of the claims 12 to 18,
**characterized in that**
a chamber (6) made of dielectric material is placed between said discharge region (3) and said first electrode (9a), said chamber (6) being filled with a gas or gas mixture which emits UV radiation in a plasma-excited state.

20. A device according to one of the claims 12 to 19,
**characterized in that**
said first electrode (4, 9a) is spaced a distance of between 0.1 to 5mm from said first side (7) of said chamber (6).

21. A device according to one of the claims 12 to 20,
**characterized in that**
said discharge region (3) is open on at least two sides.

22. A method for operating a device according to one of the claims 12 - 21,
in which between said first electrode (4, 9a) and said further electrode (9, 9b), a sparking voltage is applied which suffices to spark only a discharge in said chamber (6) but does not suffice to spark a discharge in said discharge region (3) so that objects placed in said discharge region (3) are only impinged with UV radiation from said chamber (6).

## Revendications

1. Procédé pour le traitement de surface d'objets, en particulier de matière en forme de bande ou emboutie, dans lequel la surface à traiter (2) de l'objet (1) dans un espace de décharge (3) rempli d'un premier gaz ou mélange gazeux, est exposée à une décharge à barrière, qui est produite entre une première (4) et une deuxième électrode plane (5),
**caractérisé en ce que**,
un deuxième gaz ou mélange gazeux excité par plasma est mis en oeuvre en tant que deuxième électrode (5), lequel émet un rayonnement UV, auquel la surface à traiter (2) est exposée en outre.

2. Procédé selon la revendication 1,
**caractérisé en ce que**,
le deuxième gaz ou mélange gazeux excité par plasma se trouve sous une pression d'au moins 100*10² Pa.

3. Procédé selon la revendication 1 ou 2,
**caractérisé en ce que**,
un gaz ou mélange gazeux formant des excimères dans l'état excité par un plasma est mis en oeuvre en tant que deuxième gaz ou mélange gazeux excité par un plasma.

4. Procédé selon l'une quelconque des revendications 1 à 3,
**caractérisé en ce que**,
un ou plusieurs gaz supplémentaires sont introduits dans l'espace de décharge (3), qui améliorent l'effet du traitement de surface et/ou l'homogénéité de la décharge à barrière.

5. Procédé selon la revendication 4,
**caractérisé en ce que**,
de l'hélium, de l'argon, de l'azote, de l'hydrogène, de l'oxygène, de l'ozone, du gaz à l'eau, de la vapeur d'eau, du gaz ou de la vapeur de peroxyde d'hydrogène, ou des combinaisons de ces gaz, sont introduits dans l'espace de décharge (3), en tant que gaz supplémentaires.

6. Procédé selon l'une quelconque des revendications 1 à 5,
**caractérisé en ce que**,
des tensions pulsées sont appliquées aux électrodes (4, 9).

7. Procédé selon l'une quelconque des revendications 1 à 6,
**caractérisé en ce que**,
une première électrode plane (4) est mise en oeuvre qui peut être métallique ou munie d'une couche diélectrique du côté de l'objet.

8. Procédé selon la revendication 7,
**caractérisé en ce que**,
la première (4) et la deuxième électrode (5) sont formées avec une grande surface de sorte que lors du traitement de surface d'une matière sous forme de bande, elles présentent au moins dans une dimension, une extension plus grande que la largeur de la matière sous forme de bande.

9. Procédé selon l'une quelconque des revendications 7 ou 8,
**caractérisé en ce que**,
plusieurs premières (4) et deuxièmes électrodes (5) sont disposées l'une à côté de l'autre, et/ou l'une derrière l'autre, afin de traiter simultanément une zone de surface plus grande.

10. Procédé selon l'une quelconque des revendications 1 à 9,
**caractérisé en ce que**,
l'objet (1) est déplacé à travers l'espace de décharge pendant la décharge à barrière.

11. Procédé selon l'une quelconque des revendications 1 à 10, pour nettoyer et/ou désinfecter, et/ou stériliser, et/ou activer des surfaces, en particulier pour aseptiser ou stériliser de la matière d'emballage.

12. Dispositif pour le traitement de surface d'objets plats, en particulier de matière en forme de bande ou emboutie, avec un espace de décharge (3), qui est formé entre une première électrode plane (4, 9a) et une chambre (6) remplie d'un gaz, qui est constituée d'une matière diélectrique transparente au rayonnement UV, au moins sur un premier côté (7) dirigé vers la première électrode (4, 9a),
**caractérisé en ce que**,
la chambre (6) sur un deuxième côté (8) faisant dos à la première électrode (4, 9a) est contigué à une électrode plane supplémentaire (9, 9b) ou est close par celle-ci et est remplie par un gaz ou mélange gazeux émettant un rayonnement UV dans l'état excité par un plasma.

13. Dispositif selon la revendication 12,
**caractérisé en ce que**,
la pression du gaz ou du mélange gazeux dans la chambre (6) est au moins de 100*10² Pa.

14. Dispositif selon la revendication 12 ou 13,
**caractérisé en ce que**,
la première (4, 9a) et l'électrode supplémentaire (9, 9b) ainsi que le premier (7) et le deuxième côté (8) de la chambre (6) sont formés de façon plane.

15. Dispositif selon la revendication 12 ou 13,
**caractérisé en ce que**,
la première (4, 9a) et l'électrode supplémentaire (9, 9b) ainsi que le premier (7) et le deuxième côté (8) de la chambre (6) sont formés de façon tridimensionnelle, en particulier recourbée.

16. Dispositif selon l'une quelconque des revendications 12 à 15,
**caractérisé en ce que**,
l'espace de décharge (3) est rempli ou parcouru par de l'air, de l'air humide, de l'oxygène ou de l'argon.

17. Dispositif selon l'une quelconque des revendications 12 à 16,
**caractérisé en ce que**,
la chambre (6) est remplie par un gaz ou mélange gazeux formant des excimères dans l'état excité par un plasma, par exemple Xe ou KrCl.

18. Dispositif selon l'une quelconque des revendications 12 à 17,
**caractérisé en ce que**,
la matière diélectrique de la chambre (6) est du verre de quartz, en particulier un verre de quartz synthétique.

19. Dispositif selon l'une quelconque des revendications 12 à 18,
**caractérisé en ce que**,
entre l'espace de décharge (3) et la première électrode (9a), est disposée une chambre (6) constituée d'une matière diélectrique, qui est remplie par un gaz ou mélange gazeux, qui émet un rayonnement UV dans l'état excité par un plasma.

20. Dispositif selon l'une quelconque des revendications 12 à 19,
**caractérisé en ce que**,
la première électrode (4, 9a) présente une distance comprise entre 0,1 et 5 mm la séparant du premier côté (7) de la chambre (6).

21. Dispositif selon l'une quelconque des revendications 12 à 20,
**caractérisé en ce que**,
l'espace de décharge (3) est ouvert sur au moins deux côtés.

22. Procédé pour exploiter un dispositif selon l'une quelconque des revendications 12 à 21,
dans lequel une tension d'allumage est appliquée entre la première (4, 9b) et l'électrode supplémentaire (9, 9b), laquelle tension suffit seulement à allumer une décharge dans la chambre (6), mais non pas à allumer une décharge dans l'espace de décharge (3), de sorte que des objets disposés dans l'espace de décharge (3) ne sont frappés que par du rayonnement UV issu de la chambre (6).
